(19) Europäisches Patentamt
European Patent Office
Office européen des brevets

(11) EP 4 327 919 A1

(12) EUROPEAN PATENT APPLICATION

(43) Date of publication:
**28.02.2024 Bulletin 2024/09**

(21) Application number: **23191409.4**

(22) Date of filing: **14.08.2023**

(51) International Patent Classification (IPC):
***B01D 67/00*** *(2006.01)* ***B01D 71/34*** *(2006.01)*
***B01D 71/64*** *(2006.01)* ***B01D 71/68*** *(2006.01)*

(52) Cooperative Patent Classification (CPC):
**B01D 67/0011; B01D 67/003; B01D 67/00793; B01D 71/34; B01D 71/64; B01D 71/68;**
B01D 2323/12; B01D 2323/18; B01D 2323/21; B01D 2325/021

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.08.2022 JP 2022133009**

(71) Applicant: **TOKYO OHKA KOGYO CO., LTD.**
**Kawasaki-shi, Kanagawa 211 0012 (JP)**

(72) Inventor: **MORITA, Takaaki**
**KAWASAKI-SHI, 211-0012 (JP)**

(74) Representative: **Plasseraud IP**
**66, rue de la Chaussée d'Antin**
**75440 Paris Cedex 09 (FR)**

(54) **POROUS FILM**

(57) An object is to provide a porous film which has excellent removal performance of viruses and the like and a long lifetime, a virus removal method which uses the porous film as a filter, a method for producing a virus-free product which uses the porous film as a filter and a device which includes the porous film as a filter. In a porous film including a structure of spherical pores communicating with each other, an interconnected pore is an opening of the spherical pores communicating with each other, and the pore diameter of the interconnected pore is set to 10 nm or more and 35 nm or less, and the number of spherical pores which are present between one surface of the porous film and the other surface thereof and are 50 nm or more and 200 nm or less is set to 200 or more and 1000 or less.

EP 4 327 919 A1

## Description

### BACKGROUND OF THE INVENTION

#### Field of the Invention

The present invention relates to a porous film which has a specific porous structure including a structure of spherical pores communicating with each other, a virus removal method which uses the porous film as a filter, a method for producing a virus-free product which uses the porous film as a filter and a device which includes the porous film as a filter.

#### Related Art

Conventionally, various porous films have been used for filters and the like. For example, porous films have been utilized to remove viruses and toxic components in liquids and gases.
Specifically, porous films are utilized in the medical field to remove viruses from a drug solution and the like (Patent Documents 1 and 2). Porous films are also utilized in the field of household water purifiers to remove viruses and bacteria from water (Patent Document 3).

Patent Document 1: PCT International Publication No. WO 2004035180 A1, Specification
Patent Document 2: PCT International Publication No. WO 2009141965 A1, Specification
Patent Document 3: Japanese Unexamined Patent Application, Publication No. 2019-48297

### SUMMARY OF THE INVENTION

However, porous films having high removal performance of viruses and the like generally tend to clog pores and have a short lifetime. The lifetime is the time after the porous film is first used for removing viruses and the like until its removal performance of viruses and the like drops below the desired performance. When the size of the pores is increased in order to prolong the lifetime of the porous film, a virus leakage problem occurs. In other words, the porous film has a trade-off relationship between the removal performance of viruses and the like and the lifetime. It is difficult to achieve both high removal performance of viruses and the like and a long lifetime in the porous film.
The present invention is made in view of the circumstances as described above, and an object thereof is to provide a porous film which has excellent removal performance of viruses and the like and a long lifetime, a virus removal method which uses the porous film as a filter, a method for producing a virus-free product which uses the porous film as a filter and a device which includes the porous film as a filter.
In a porous film including a structure of spherical pores communicating with each other, an interconnected pore is an opening of the spherical pores communicating with each other, and the pore diameter of the interconnected pore is set to 10 nm or more and 35 nm or less, and the number of spherical pores which are present between one surface of the porous film and the other surface thereof and are 50 nm or more and 200 nm or less is set to 200 or more and 1000 or less, with the result that the present inventors have found that the problem described above is solved to achieve the present invention. Specifically, the followings are provided.
According to a first aspect of the present invention, a porous film is provided which includes: a structure of spherical pores communicating with each other,
an interconnected pore is an opening of the spherical pores communicating with each other, and the pore diameter of the interconnected pore is 10 nm or more and 35 nm or less and the number of the spherical pores which are present between one surface of the porous film and the other surface thereof and are 50 nm or more and 200 nm or less is 200 or more and 1000 or less.
According to a second aspect of the present invention, a virus removal method is provided which includes: using the porous film according to the first aspect as a filter to remove, from a gas or a liquid containing viruses, the viruses.
According to a third aspect of the present invention, a method for producing a virus-free sample or a virus-free product is provided, and the method includes: using the porous film according to the first aspect as a filter to remove, from a liquid, a gaseous sample or a product containing viruses, the viruses.
According to a fourth aspect of the present invention, a device is provided which includes: a function of removing, from a gas or a liquid containing viruses, the viruses; and the porous film according to the first aspect serving as a virus removal filter.
According to the present invention, it is possible to provide a porous film which has excellent removal performance of viruses and the like and a long lifetime, a virus removal method which uses the porous film as a filter, a method for producing a virus-free product which uses the porous film as a filter and a device which includes the porous film as

a filter.

DETAILED DESCRIPTION OF THE INVENTION

<<Porous film>>

A porous film includes a structure (hereinafter referred to as an interconnected pore for short) of spherical pores communicating with each other. When the porous film is a multilayer body, the same is true for porous layers included in the multilayer body.

The spherical shape regarding the shape of the pore is a concept which includes a true spherical shape. However, the spherical shape is not necessarily limited to only the true spherical shape. Preferably, the spherical shape is a substantially true spherical shape. The spherical shape includes a shape which can be recognized as a substantially true spherical shape when an enlarged image of the pore is visually checked. Specifically, in the spherical pore, a surface which defines the pore is a curved surface. The pore in a true spherical shape or a substantially true spherical shape is preferably defined by the curved surface. When the porous film is the multilayer body, a porosity and the pore diameter of the spherical pores which make up the interconnected pore may be the same as or different from each other in the porous layers of the multilayer body.

In the porous film, individual spherical pores are typically pores which are formed by removing, in a subsequent step, individual fine particles present in a resin-fine particle composite film which will be described later. The interconnected pore is formed by removing, in a subsequent step, a plurality of fine particles which are present in contact with each other in the resin-fine particle composite film in a method for producing the porous film which will be described later. The parts where the spherical pores communicate with each other in the interconnected pore are derived from the parts where a plurality of fine particles are in contact with each other before being removed.

The diameter of the spherical pore is preferably 40 nm or more and 300 nm or less, more preferably 50 nm or more and 200 nm or less, further preferably 50 nm or more and 100 nm or less and particularly preferably 80 nm or more and 100 nm or less. In the case of a virus removal application, the diameter of the interconnected pore is preferably 10 nm or more and 35 nm or less, more preferably 15 nm or more and 30 nm or less and further preferably 20 nm or more and 30 nm or less. The average diameter of the spherical pore is preferably 1.1 times or more and 20 times or less the average diameter of the interconnected pore, more preferably 1.5 times or more and 10 times or less the average diameter of the interconnected pore and further preferably 2 times or more and 6 times or less the average diameter of the interconnected pore. The number of spherical pores which are present between one surface of the porous film and the other surface thereof and are 50 nm or more and 200 nm or less is preferably 200 or more and 1100 or less, more preferably 200 or more and 1000 or less and further preferably 200 or more and 700 or less.

The porous film includes, therewithin, the interconnected pore which penetrates the porous film in a thickness direction and serves as a flow path of a fluid. In this way, the fluid can permeate from one main surface of the porous film to the other main surface thereof. When the porous film is used as a filter, the fluid passes through the interior of the porous film in contact with curved surfaces which define the individual spherical pores.

In the present specification, when the porous film is subjected to chemical etching treatment which will be described later, the pore diameter can be measured by a porometer (bubble point method). The porometer is used to determine the amount of variation in the size of the interconnected pore before and after the etching treatment, and the actual average pore diameter can be determined from the amount of variation.

A size of the interconnected pore can be obtained by using the porometer according to the following formula.

$$d = C\gamma/P$$

Herein, d is the size of the interconnected pore (μm). $\gamma$ is surface tension of a liquid (mN/m). P is pressure (kg/cm$^2$). C is a constant. The constant C is 0.415 in a case in which a unit for pressure is psi. When air pressure is applied to the porous film wetted with the liquid, air passes when the applied air pressure is increased by the capillary tension of the liquid in the pores. In the bubble point method, the principle is utilized that a smaller pore (for example, the interconnected pore) requires a higher pressure in order to allow air to pass therethrough. By comparing gas flow rates for both wet and dry porous films at the same pressure, the percentage of a flow rate passing through the pores of a specified size or larger in the porous film can be calculated from a relationship between the pressure and the pore size. At the same time, by gradually increasing the pressure, the flow rate distribution of very small pore size increments (about 10 nm) can also be determined by the difference.

Based on data obtained in this way, a pressure at which the flow rate of air in the wet porous film is half of the flow rate of air in the dry porous film is determined, and then the pore diameter at that time is determined by the formula described above and is assumed to be the average size of the interconnected pore.

The pore diameter of the spherical pores included in the porous film is calculated from a three-dimensional

image acquired by shooting a cross-sectional image of the porous film using an FIB-SEM (focused ion beam scanning microscope). On one main surface of the porous film, an arbitrary region having a size of 5 μm × 5 μm is first defined. Cross-sectional SEM images of the porous film are acquired at intervals of 2 nm over the entire thickness range of the porous film while cutting is being performed in the range of 5 μm × 5 μm by 2 nm in a film thickness direction with the FIB. The acquired cross-sectional SEM images are three-dimensionally connected together to create a three-dimensional image of film thickness × 5 μm × 5 μm. In the three-dimensional image obtained, the diameters of 100 spherical pores are measured, and the average value of the measured values of the 100 diameters is used as the pore diameter of the spherical pore.

The number of spherical pores included in a direction perpendicular to the film thickness of the porous film is calculated from the number of spherical pores in a three-dimensional image acquired by shooting a cross-sectional image of the porous film using the FIB-SEM. On one main surface of the porous film, an arbitrary region having a size of 5 μm × 5 μm is first defined. Cross-sectional SEM images of the porous film are acquired at intervals of 2 nm over the entire film thickness range of the porous film while cutting is being performed in the range of 5 μm × 5 μm by 2 nm in the film thickness direction with the FIB. A three-dimensional image of film thickness × 5 μm × 5 μm is created from the acquired cross-sectional SEM images.

100 three-dimensional images of film thickness × spherical pore diameter × spherical pore diameter are cut out from the three-dimensional image, and the number of spherical pores in each of the three-dimensional images is calculated. The number of incomplete spherical pores included in the three-dimensional image is not included in the number of spherical pores. The incomplete spherical pore is, for example, a hemispherical pore. The average value of the numbers of spherical pores in the 100 three-dimensional images is assumed to be the number of spherical pores in the direction perpendicular to the film thickness. The number of spherical pores in the direction perpendicular to the film thickness is the number of spherical pores which are present between one surface of the porous film and the other surface thereof.

The film thickness of the entire porous film is not particularly limited. The film thickness of the entire porous film can be adjusted as necessary according to the diameter of the spherical pore. For example, when the porous film is used as a filter or the like, the film thickness of the entire porous film is preferably 15 μm or more and 100 μm or less, more preferably 18 μm or more and 80 μm or less and further preferably 18 μm or more and 70 μm or less. The above film thickness can be determined by measuring the thicknesses of a plurality of parts with, for example, a micrometer and averaging the thicknesses.

A material for forming the porous film is not particularly limited, and may be an organic material or an inorganic material. A polymeric material is typically preferable due to its excellent workability and flexibility. Examples thereof include various resin solutions, thermosetting resin compositions and photosensitive resin compositions. As the photosensitive resin composition, there are a positive composition in which an exposed portion is soluble in a developer and a negative composition in which an exposed portion is insoluble in a developer, and both of them can be used for forming the porous film. As the material for forming the porous film, the thermosetting resin composition which is cured by heating is preferable in that a thin film having excellent strength can be formed.

<Varnish for producing porous film>

In the production of the porous film described above, a varnish for producing a porous film (hereinafter also simply referred to as the "varnish") is used which contains fine particles, a resin and a solvent that are predetermined and in which the resin is dissolved in the solvent. The varnish is typically produced by: preparing a fine particle dispersion liquid to disperse the fine particles in the solvent; preparing a resin solution including at least one resin selected from the group consisting of polyvinylidene fluoride, polyethersulfone, polyamide acid, polyimide, polyamide acid serving as a polyamidoimide precursor and polyamidoimide; and kneading in which the fine particle dispersion liquid and the resin solution are combined and kneaded to adjust its concentration.

For kneading the varnish, a rotation/revolution mixer (for example, product name: Awatori Rentaro made by Thinky Corporation), a planetary mixer, a bead mill or the like can be used.

[Resin]

As described above, the varnish includes at least one resin selected from the group consisting of polyvinylidene fluoride, polyethersulfone, polyamide acid, polyimide, a polyamidoimide precursor and polyamidoimide. These resins will be described below.

(Polyvinylidene fluoride)

The polyvinylidene fluoride is not particularly limited as long as it can be dissolved in the solvent used for formation of the varnish. The polyvinylidene fluoride may be a homopolymer or may be a copolymer. Examples of

structural units to be copolymerized include ethylene, ethylene trifluoride, ethylene tetrafluoride, propylene hexafluoride and the like, and the mass average molecular weight thereof is, for example, about ten thousand or more and five million or less.

(Polyethersulfone)

Polyethersulfone is not particularly limited as long as it can be dissolved in the solvent used for formation of the varnish. Polyethersulfone can be appropriately selected depending on the use of porous film produced and it may be hydrophilic or hydrophobic. Furthermore, it may be aliphatic polyethersulfone or aromatic polyethersulfone. The mass average molecular weight is, for example, 5,000 or more and 1,000,000 or less, and preferably 10,000 or more and 300,000 or less.

(Polyamide acid)

The polyamide acid may be any product prepared by polymerizing appropriate tetracarboxylic dianhydride and diamine, which can be used without particular limitation. The amounts of the tetracarboxylic dianhydride and the diamine to be used are not particularly limited, and the amount of the diamine is preferably 0.50 or more and 1.50 mol or less, more preferably 0.60 or more and 1.30 mol or less, and particularly preferably 0.70 or more and 1.20 mol or less, based on 1 mol of the tetracarboxylic dianhydride.

The tetracarboxylic dianhydride can be appropriately selected from tetracarboxylic dianhydrides that have been conventionally used as raw materials for synthesizing polyamide acids. The tetracarboxylic dianhydride may be an aromatic tetracarboxylic dianhydride or an aliphatic tetracarboxylic dianhydride, but from the viewpoint of the heat resistance of the resulting polyimide resin, an aromatic tetracarboxylic dianhydride is preferably used. One type of tetracarboxylic dianhydride may be used alone or in combination of two or more types thereof.

Preferred examples of the aromatic tetracarboxylic dianhydride include pyromellitic dianhydride, 1,1-bis(2,3-dicarboxyphenyl)ethane dianhydride, bis(2,3-dicarboxyphenyl)methane dianhydride, bis(3,4-dicarboxyphenyl)methane dianhydride, 3,3',4,4'-biphenyltetracarboxylic dianhydride, 2,3,3',4'-biphenyltetracarboxylic dianhydride, 2,2,6,6-biphenyltetracarboxylic dianhydride, 2,2-bis(3,4-dicarboxyphenyl)propane dianhydride, 2,2-bis(2,3-dicarboxyphenyl)propane dianhydride, 2,2-bis(3,4-dicarboxyphenyl)-1,1,1,3,3,3-hexafluoropropane dianhydride, 2,2-bis (2,3-dicarboxyphenyl)-1,1,1,3,3,3-hexafluoropropane dianhydride, 3,3',4,4'-benzophenonetetracarboxylic dianhydride, bis(3,4-dicarboxyphenyl)ether dianhydride, bis(2,3-dicarboxyphenyl)ether dianhydride, 2,2',3,3'-benzophenonetetracarboxylic dianhydride, 4,4-(p-phenylenedioxy)diphthalic dianhydride, 4,4-(m-phenylenedioxy)diphthalic dianhydride, 1,2,5,6-naphthalenetetracarboxylic dianhydride, 1,4,5,8-naphthalenetetracarboxylic dianhydride, 2,3,6,7-naphthalenetetracarboxylic dianhydride, 1,2,3,4-benzenetetracarboxylic dianhydride, 3,4,9,10-perylenetetracarboxylic dianhydride, 2,3,6,7-anthracenetetracarboxylic dianhydride, 1,2,7,8-phenanthrenetetracarboxylic dianhydride, 9,9-bisphthalic anhydride fluorene, and 3,3',4,4'-diphenylsulfonetetracarboxylic dianhydride. Examples of the aliphatic tetracarboxylic dianhydride include ethylenetetracarboxylic dianhydride, butanetetracarboxylic dianhydride, cyclopentanetetracarboxylic dianhydride, cyclohexanetetracarboxylic dianhydride, 1,2,4,5-cyclohexanetetracarboxylic dianhydride, and 1,2,3,4-cyclohexanetetracarboxylic dianhydride. Among these, 3,3',4,4'-biphenyltetracarboxylic dianhydride and pyromellitic dianhydride are preferred because of their inexpensiveness, ready availability, etc. One type of tetracarboxylic dianhydride may be used alone or as a mixture of two or more thereof.

The diamine can be appropriately selected from diamines that have been conventionally used as raw materials for synthesizing polyamide acids. The diamine may be an aromatic diamine or an aliphatic diamine, but from the viewpoint of the heat resistance of the resulting polyimide resin, an aromatic diamine is preferred. One type of these diamines may be used alone or in combination of two or more types thereof.

Examples of the aromatic diamine include diamino compounds having one phenyl group or about two or more and ten or less phenyl groups. Specifically, examples of the aromatic diamine include phenylenediamines and their derivatives, diaminobiphenyl compounds and their derivatives, diaminodiphenyl compounds and their derivatives, diaminotriphenyl compounds and their derivatives, diaminonaphthalenes and their derivatives, aminophenylaminoindanes and their derivatives, diaminotetraphenyl compounds and their derivatives, diaminohexaphenyl compounds and their derivatives, and cardo-type fluorenediamine derivatives.

The phenylenediamines are, for example, m-phenylenediamine and p-phenylenediamine. The phenylenediamine derivatives are diamines to which alkyl groups, such as methyl group and ethyl group, are bound, such as 2,4-diaminotoluene and 2,4-triphenylenediamine.

In the diaminodiphenyl compounds, two aminophenyl groups are bonded to each other. For example, the diaminodiphenyl compounds are 4,4'-diaminobiphenyl and 4,4'-diamino-2,2'-bis(trifluoromethyl)biphenyl.

The diaminodiphenyl compound is a compound obtained by linkage of two aminophenyl groups at their phenyl groups via another group. The linkage is, for example, an ether linkage, a sulfonyl linkage, a thioether linkage, a linkage

of an alkylene or its derivative group, an imino linkage, an azo linkage, a phosphine oxide linkage, an amide linkage, or an ureylene linkage. The number of carbon atoms of the alkylene linkage is about 1 or more and 6 or less. The derivative group is an alkylene group whose one or more hydrogen atoms have been replaced by, for example, halogen atoms.

Examples of the diaminodiphenyl compounds include 3,3'-diaminodiphenyl ether, 3,4'-diaminodiphenyl ether, 4,4'-diaminodiphenyl ether, 3,3'-diaminodiphenyl sulfone, 3,4'-diaminodiphenyl sulfone, 4,4'-diaminodiphenyl sulfone, 3,3'-diaminodiphenyl methane, 3,4'-diaminodiphenyl methane, 4,4'-diaminodiphenyl methane, 4,4'-diaminodiphenyl sulfide, 3,3'-diaminodiphenyl ketone, 3,4'-diaminodiphenyl ketone, 2,2-bis(p-aminophenyl)propane, 2,2'-bis(p-aminophenyl)hexafluoropropane, 4-methyl-2,4-bis(p-aminophenyl)-1-pentene, 4-methyl-2,4-bis(p-aminophenyl)-2-pentene, iminodianiline, 4-methyl-2,4-bis(p-aminophenyl)pentane, bis(p-aminophenyl)phosphine oxide, 4,4'-diaminoazobenzene, 4,4'-diaminodiphenylurea, 4,4'-diaminodiphenylamide, 1,4-bis(4-aminophenoxy)benzene, 1,3-bis(4-aminophenoxy)benzene, 1,3-bis(3-aminophenoxy)benzene, 4,4'-bis(4-aminophenoxy)biphenyl, bis[4-(4-aminophenoxy)phenyl] sulfone, bis[4-(3-aminophenoxy)phenyl] sulfone, 2,2-bis[4-(4-aminophenoxy)phenyl] propane, and 2,2-bis[4-(4-aminophenoxy)phenyl]hexafluoropropane.

Among these, p-phenylenediamine, m-phenylenediamine, 2,4-diaminotoluene, and 4,4'-diaminodiphenylether are preferred because of their inexpensiveness, ready availability, etc.

The diaminotriphenyl compound is formed by linkage of two aminophenyl groups and one phenylene group, all of which are each linked through another group. The "another group" is selected from the same groups as in the diaminodiphenyl compounds. Examples of the diaminotriphenyl compounds include 1,3-bis(m-aminophenoxy)benzene, 1,3-bis(p-aminophenoxy)benzene, and 1,4-bis(p-aminophenoxy)benzene.

Examples of the diaminonaphthalenes include 1,5-diaminonaphthalene and 2,6-diaminonaphthalene.

Examples of the aminophenylaminoindanes include 5- or 6-amino-1-(p-aminophenyl)-1,3,3-trimethylindane.

Examples of the diaminotetraphenyl compounds include 4,4'-bis(p-aminophenoxy)biphenyl, 2,2'-bis[p-(p'-aminophenoxy)phenyl]propane, 2,2'-bis[p-(p'-aminophenoxy)biphenyl]propane, and 2,2'-bis[p-(m-aminophenoxy)phenyl] benzophenone.

An example of the cardo-type fluorenediamine derivatives is 9,9-bisanilinefluorene.

The number of carbon atoms of aliphatic diamine is, for example, about 2 or more and 15 or less. Specific examples of aliphatic diamine include pentamethylenediamine, hexamethylenediamine, and heptamethylenediamine.

Note here that hydrogen atoms of these diamines may be a compound having at least one substituent selected from the group consisting of halogen atoms and methyl, methoxy, cyano, and phenyl groups.

There is no particular limitation to means for producing the polyamide acid, and, for example, well-known technique such as a method for reacting an acid and a diamine component in a solvent can be used.

The reaction of a tetracarboxylic dianhydride and a diamine is usually performed in a solvent. The solvent to be used for the reaction of a tetracarboxylic dianhydride and a diamine is not particularly limited and may be any solvents that can dissolve the tetracarboxylic dianhydride and the diamine without reacting with the tetracarboxylic dianhydride and the diamine. One type of solvent may be used alone or in combination of two or more types thereof.

Examples of the solvent to be used for the reaction of a tetracarboxylic dianhydride and a diamine include nitrogen-containing polar solvents, such as N-methyl-2-pyrrolidone, N,N-dimethyl acetamide, N,N-diethylacetamide, N,N-dimethylformamide, N,N-diethylformamide, N-methylcaprolactam, and N,N,N',N'-tetramethylurea; lactone polar solvents, such as β-propiolactone, γ-butyrolactone, γ-valerolactone, δ-valerolactone, γ-caprolactone, and ε-caprolactone; dimethyl sulfoxide; acetonitrile; fatty acid esters, such as ethyl lactate and butyl lactate; ethers, such as diethylene glycol dimethyl ether, diethylene glycol diethyl ether, dioxane, tetrahydrofuran, methyl cellosolve acetate, and ethyl cellosolve acetate; and phenol solvents, such as cresols and xylene-based solvent. One type of these solvents may be used alone or in combination of two or more types thereof. The amount of the solvent to be used is not particularly limited but is desirably such that the content of the resulting polyamide acid is 5% or more and 50% by mass or less.

Among these solvents, from the viewpoint of the solubility of the resulting polyamide acid, preferred are nitrogen-containing polar solvents, such as N-methyl-2-pyrrolidone, N,N-dimethyl acetamide, N,N-diethylacetamide, N,N-dimethylformamide, N,N-diethylformamide, N-methylcaprolactam, and N,N,N',N'-tetramethylurea.

The polymerization temperature is usually -10°C or more and 120°C or less and preferably 5°C or more and 30 °C or less. The polymerization time varies depending on the raw material composition to be used, and is usually 3 hours or more and 24 hours or less. One type of the polyamide acid may be used alone or in combination of two or more types thereof.

(Polyimide)

The polyimide can be any known polyimide, and used without any limitation to its structure and molecular weight. The side chain of the polyimide may have a condensable functional group, such as a carboxy group, or a functional group enhancing the cross-linking reaction, etc. during calcining. Furthermore, when the varnish contains a solvent, polyimide that can be solved in a solvent used is preferable.

In order to make the polyimide soluble in a solvent, it is effective to use a monomer for introducing a flexible bend structure into the main chain, for example, to use an aliphatic diamine, such as ethylenediamine, hexamethylenediamine, 1,4-diaminocyclohexane, 1,3-diaminocyclohexane, and 4,4'-diaminodicyclohexylmethane; an aromatic diamine, such as 2-methyl-1,4-phenylenediamine, o-tolidine, m-tolidine, 3,3'-dimethoxybenzidine, and 4,4'-diaminobenzanilide; a polyoxyalkylenediamine, such as polyoxyethylenediamine, polyoxypropylenediamine, and polyoxybutyrenediamine; a polysiloxanediamine; 2,3,3',4'-oxydiphthalic anhydride, 3,4,3',4'-oxydiphthalic anhydride, and 2,2-bis(4-hydroxyphenyl)propanedibenzoate-3,3',4,4'-tetracarboxylic dianhydride. It is also effective to use a monomer containing a functional group for improving the solubility in a solvent, for example, to use a fluorinated diamine, such as 2,2'-bis(trifluoromethyl)-4,4'-diaminobiphenyl and 2-trifluoromethyl-1,4-phenylenediamine. Furthermore, in addition to the monomer for improving the solubility of the polyimide, a monomer that is mentioned in the paragraph describing the polyamide acid may be used within a range that does not inhibit the solubility. Each of polyimide and the monomer thereof may be used alone or may be used in combination of two or more types thereof.

There is no limitation to means for producing polyimide. Polyimide may be produced by any well-known techniques, for example, chemically imidizing or thermally imidizing polyamide acid. Examples of such polyimides include aliphatic polyimide (full-aliphatic polyimides) and aromatic polyimides, and aromatic polyimides are preferable. The aromatic polyimide may be one prepared by a thermal or chemical ring-closing reaction of a polyamide acid having repeating units represented by Formula (1) or a polyimide having repeating units represented by Formula (2). In the formulae, Ar represents an aryl group. When the varnish contains a solvent, these polyimides may be then solved in a solvent to be used.

[Chem. 1]

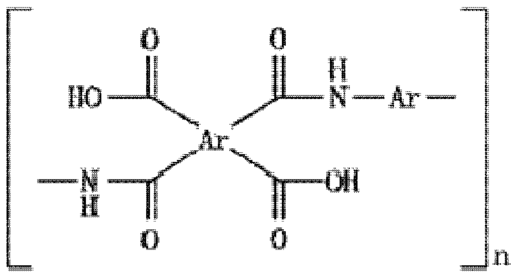
····(1)

[Chem. 2]

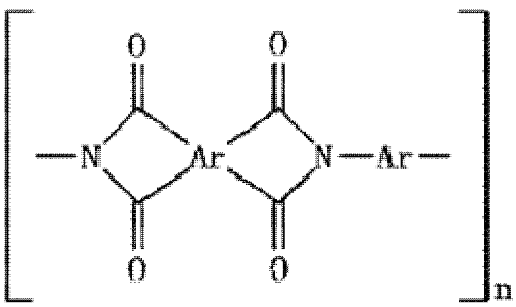
····(2)

(polyamidoimide and polyamidoimide precursor)

Any well-known polyamidoimides can be used without limitation to the structure or molecular weight. The side chain of the polyamidoimide may have a condensable functional group, such as a carboxy group, or a functional group enhancing the cross-linking reaction, etc. during calcining. Furthermore, when the varnish contains a solvent, a soluble polyamidoimide that can be solved in a solvent to be used is preferable.

As the polyamidoimide, (i) a resin obtained by reacting an acid having a carboxyl group and an acid anhydride group with diisocyanate in one molecule of trimellitic anhydride and the like, (ii) a resin obtained by imidization of a precursor polymer obtained by reacting a reactive derivative of the acid such as trimellitic anhydride chloride and diamine (a polyamide-imide precursor) can be usually used without particular limitation.

Examples of the above-mentioned acids or the reactive derivatives include trimellitic anhydride, trimellitic anhydride halides such as trimellitic anhydride chloride, trimellitic anhydride esters, and the like.

Examples of the above-mentioned optional diamine include diamines described as an example in the description of the above-mentioned polyamide acid. A diaminopyridine compound can also be used.

The above mentioned any diisocyanate is not particularly limited, and includes, for example, a diisocyanate compound corresponding to the above-mentioned optional diamine can be used. Specific examples thereof include meta-phenylene diisocyanate, p-phenylene diisocyanate, o-tolidine diisocyanate, p-phenylene diisocyanate, m-phenylene diisocyanate, 4,4'-oxybis (phenyl isocyanate), 4,4'-diphenylmethane diisocyanate, bis[4-(4-isocyanate phenoxy) phenyl] sulfone, 2,2'-bis[4-(4-isocyanate phenoxy) phenyl] propane, 2,4-tolylene diisocyanate, 2,6-tolylene diisocyanate, 4,4'-diphenylmethane diisocyanate, 3,3'-dimethyldiphenyl-4,4'-diisocyanate, 3,3'-diethyldiphenyl-4,4'-diisocyanate, isophorone diisocyanate, hexamethylene diisocyanate, 4,4'-dicyclohexyl methane diisocyanate, m-xylene diisocyanate, p-xylene diisocyanate, naphtalen diisocyanate, and the like, can be exemplified.

As a raw material monomer of polyamidoimide, in addition to the above, it is possible to use compounds described as general formulae in Japanese Unexamined Patent Application, Publication No. S63-283705 and Japanese Unexamined Patent Application, Publication No. H2-198619. Furthermore, the imidization in the method described in the above (ii) may be any one of thermal imidization and chemical imidization. As the chemical imidization, a method of immersing an uncalcined composite film formed by using a varnish such as a polyamidoimide precursor in a mixed solvent including acetic anhydride, or acetic anhydride and isoquinoline, and the like can be used. Note here that the polyamidoimide precursor can be referred to as a polyimide precursor from the viewpoint that it is a precursor before imidization.

The polyamidoimide to be contained in a varnish may be the above-mentioned (1) a polymer obtained by reacting an acid such as trimellitic anhydride and diisocyanate with each other, (2) a polymer obtained by imidization of a precursor polymer obtained by reacting a reactive derivative of the above-mentioned acid such as trimellitic anhydride and diamine with each other, or the like. The term "polyamidoimide precursor" in this specification and claims means a polymer before imidization (a precursor polymer). Each of polyamidoimide and polyamidoimide precursor may be used alone or in combination of two or more types thereof. Furthermore, as the polyamidoimide, each of the above-mentioned polymer, raw material monomer, and oligomer may be used alone or in combination of two or more types thereof.

(Fine particles)

The material of the fine particles is not particularly limited and may adopt any known material as long as the material is insoluble in the solvent contained in the varnish and can be removed later from resin-fine particle composite film. Examples of the inorganic material include metal oxides, such as silica (silicon dioxide), titanium oxide, and alumina ($Al_2O_3$). Examples of the organic materials include high-molecular-weight olefins (such as polypropylene and polyethylene) and organic polymer fine particles, such as polystyrenes, epoxy resins, celluloses, polyvinyl alcohols, polyvinyl butyrals, polyesters, and polyethers.

Specific examples of the fine particles include colloidal silica. In particular, it is preferable to select monodisperse spherical silica particles because uniform pores can be formed.

The fine particles preferably have a high sphericity and a small particle size distribution index. The fine particles satisfying these conditions are excellent in dispersibility in the varnish, and can be used without agglomeration. The average particle size of the fine particles used is preferably, for example, 50 nm or more and 200 nm or less. These conditions are satisfied, and thus the pore diameter of the porous film obtained by removing the fine particles can be made uniform. The fine particles may be used alone or may be used in combination of two or more types.

[Solvent]

The solvent is not particularly limited as long as it can dissolve the resin described above and does not dissolve the fine particles. Examples of the solvent include the solvents exemplified as the solvent used for the reaction of tetracarboxylic dianhydride and diamine. The solvent may be used alone or may be used in combination of two or more types. In the case of the polyvinylidene fluoride, examples of the solvent include, in addition to the above mentioned nitrogen-containing polar solvents, lower alkyl ketones such as methyl ethyl ketone, acetone and tetrahydrofuran, trimethyl phosphate and the like. In the case of the polyethersulfone, in addition to the above mentioned nitrogen-containing polar solvents, polar solvents such as diphenylsulfone, dimethylsulfone, dimethylsulfoxide, benzophenone, tetrahydrothiophene-1,1-dioxide, 1,3-dimethyl-2-imidazolidinone and the like.

[Dispersant]

In order to uniformly disperse the fine particles in the varnish, a dispersant may be further added together with the fine particles. The dispersant is added, and thus the fine particles can be more uniformly mixed in the varnish, and

furthermore, the fine particles can be uniformly distributed in a film including the varnish. Consequently, dense openings can be provided in the surface of the porous film which is finally obtained, and thus the front and back surfaces can be made to efficiently communicate with each other, with the result that the air permeability of the porous film is enhanced. Furthermore, the dispersant is added, and thus the drying property of the varnish is easily enhanced, and the separation of the uncalcined composite film formed from a substrate or the like is easily enhanced.

The dispersant is not particularly limited and any known dispersant may be used. Examples of the dispersant include, but not limited to, anionic surfactants, such as salts of coconut fatty acid, salts of sulfonated castor oil, lauryl sulfate, polyoxyalkylene allylphenyl ether sulfate, alkylbenzenesulfonic acid, alkylbenzene sulfonate, alkyldiphenyl ether disulfonate, alkylnaphthalene sulfonate, dialkyl sulfosuccinate, isopropyl phosphate, polyoxyethylene alkyl ether phosphate, and polyoxyethylene allylphenyl ether phosphate; cationic surfactants, such as oleylamine acetate, lauryl pyridinium chloride, cetyl pyridinium chloride, lauryl trimethylammonium chloride, stearyl trimethylammonium chloride, behenyl trimethylammonium chloride, and didecyl dimethylammonium chloride; amphoteric surfactants, such as coconut alkyl dimethylamine oxide, fatty acid amide propyl dimethyl amine oxide, alkyl polyaminoethyl glycine hydrochloride, amide betaine surfactant, alanine surfactant, and lauryl iminodipropionic acid; polyoxyalkylene primary alkyl ether or polyoxyalkylene secondary alkyl ether nonionic surfactants, such as polyoxyethylene octyl ether, polyoxyethylene decyl ether, polyoxyethylene lauryl ether, polyoxyethylene laurylamine, polyoxyethylene oleylamine, polyoxyethylene polystyryl phenyl ether, and polyoxyalkylene polystyryl phenyl ether; other polyoxyalkylene nonionic surfactants, such as polyoxyethylene dilaurate, polyoxyethylene laurate, polyoxyethylenated castor oil, polyoxyethylenated hydrogenated castor oil, sorbitan laurate, polyoxyethylene sorbitan laurate, and fatty acid diethanolamide; fatty acid alkyl esters, such as octyl stearate and trimethylolpropane tridecanoate; and polyether polyols, such as polyoxyalkylene butyl ether, polyoxyalkylene oleyl ether, and trimethylol propane tris(polyoxyalkylene) ether. These dispersants may be used as a mixture of two or more thereof.

From the view point of, for example, the film formability, the content of the dispersant in the varnish is preferably 0.01% by mass or more and 5% by mass or less, more preferably 0.05% by mass or more and 1% by mass or less, and further more preferably 0.1% by mass or more and 0.5% by mass or less, with respect to the fine particles.

[Preferred method for producing porous film]

[Step of forming uncalcined composite film]

In a step of forming the uncalcined composite film, for example, the varnish described above is applied on the substrate, the varnish is dried at 0°C or higher and 100°C or lower under normal pressure or vacuum, and preferably at 10°C or higher and 100°C or lower under normal pressure and thus it is possible to form the uncalcined composite film. Examples of the substrate includes a PET film, a SUS stainless steel substrate, a glass substrate, and the like.

When the uncalcined composite film is peeled from the substrate, the substrate provided with a mold release layer in advance can also be used in order to further enhance the releasability of the film. In a case of providing a mold release layer in the substrate in advance, the mold release agent is applied onto the substrate and is dried or baked before the application of the varnish. The mold release agent used here may be a known mold release agent, such as an alkylphosphate ammonium salt-based or fluorine-based agent or silicon, without particular restrictions. When the dried uncalcined composite film is peeled from the substrate, a slight amount of the mold release agent remains on the surface of the peeled uncalcined composite film and may lead to discoloration during calcining and adverse effects on the electrical characteristics, and the mold release agent should therefore be removed as much as possible. In order to remove the mold release agent, a washing step of washing the uncalcined composite film peeled from the substrate with an organic solvent may be introduced.

Alternatively, when the substrate is directly used, as it is, without providing a mold release layer in formation of the uncalcined composite film, the step of forming the mold release layer and the washing step can be omitted. Furthermore, in the producing an uncalcined composite film, before the below-mentioned calcining step, an immersion step into a water-containing solvent, a pressing step, and a drying step after the immersion step may be optionally provided.

[calcining step]

Post-treatment (calcination) by heating is performed on the uncalcined composite film to form the composite film (resin-fine particle composite film) composed of the resin and the fine particles. The calcining temperature in the calcining step varies depending on the structures of the uncalcined composite film and the presence or absence of a condensing agent, but the temperature is preferably 120°C or more and 450°C or less, and more preferably 150°C or more and 400°C or less. In a case of using an organic material for the fine particles, the calcining temperature need to be set to a temperature lower than the thermal decomposition temperature of the organic material. In the calcining step,

imidization is preferably completed.

The calcining can be performed by, for example, a method of increasing the temperature from room temperature to 400°C over three hours and then holding 400°C for 20 minutes or a method of stepwise drying-thermal imidization by stepwise increasing the temperature by 50°C from room temperature to 400°C (holding the temperature of each step for 20 minutes) and finally holding 400°C for 20 minutes. When the uncalcined composite film is formed on the substrate and the uncalcined composite film is peeled from the substrate once, an end of the uncalcined composite film may be fixed to, for example, a frame made of SUS stainless steel to prevent deformation.

[Fine particle-removing step]

The fine particles are removed from the resin-fine particle composite film formed as described above by a selected appropriate method, and thus it is possible to produce the porous film of a desired structure with good reproducibility. For example, when silica is used as the material of the fine particles, the resin-fine particle composite film is treated with a low-concentration hydrogen fluoride solution or the like, and thus the silica can be dissolved and removed. When the fine particles are organic fine particles, the organic fine particles are thermally decomposed, and thus the fine particles can also be removed from a polyimide resin-fine particle composite film. When the fine particles are organic fine particles, a treatment liquid which dissolves the fine particles but does not dissolve the resin is selected, treatment is performed with the treatment liquid and thus it is possible to remove the organic fine particles. As the treatment liquid, an organic solvent is typically used. When the organic fine particles are soluble in acid or alkali, an acidic aqueous solution or an alkaline aqueous solution can also be used as the treatment liquid.

[Resin-removing step]

A method for producing a porous film may include a resin-removing step of removing at least a part of a resin portion of a resin-fine particle composite film before the fine particle-removing step, or removing at least a part of the porous film after the fine particle-removing step. Before the fine particle-removing step, at least a part of a resin portion of the resin-fine particle composite film is removed or after the fine particle-removing step, at least a part of the porous film is removed, and thus as compared with a case where the removal is not performed, it is possible to enhance the porosity of the porous film serving as the final product.

The step of removing at least a part of the resin portion or the step of removing at least a part of the porous film can be carried out by a usual chemical etching or physical removing method, or a method combining these methods.

The chemical etching method includes treatment using a chemical etchant such as an inorganic alkaline solution or an organic alkaline solution. An inorganic alkaline solution is preferable. Examples of the inorganic alkaline solution include a hydrazine solution including hydrazine hydrate and ethylenediamine; a solution of alkaline metal hydroxide such as potassium hydroxide, sodium hydroxide, sodium carbonate, sodium silicate, and sodium metasilicate; an ammonium solution; an etchant including alkali hydroxide, hydrazine, and 1,3-dimethyl-2-imidazolidinone as a main component, or the like. Examples of the organic alkaline solution include an alkaline solution of primary amines such as ethyl amine and n-propyl amine; secondary amines such as diethyl amine and di-n-butylamine; tertiary amines such as triethylamine and methyl diethyl amine; alcohol amines such as dimethyl ethanol amine and triethanolamine; quaternary ammonium salts such as tetramethylammonium hydroxide and tetraethylammonium hydroxide; cyclic amines such as pyrrole and piperidine, or the like.

As a solvent for each solution, pure water and alcohols can be appropriately selected. Furthermore, solvents in which an appropriate amount of surfactant is added can be used. An alkali concentration is, for example, 0.01% by mass or more and 20% by mass or less.

Furthermore, examples of the physical method include dry etching by plasma (oxygen, argon, etc.), corona discharge, or the like, a method for treating a surface of a film by dispersing abrasives (for example, alumina (rigidity 9), or the like) in a liquid and irradiating the surface of a film with the liquid at the irradiation rate of 30 m/s or more and 100 m/s or less, and the like.

The above-mentioned methods are preferable because they are applicable in the resin-removing step both before and after the fine particle-removing step.

On the other hand, as the physical method that can be applied only to the resin-removing step carried out after the fine particle-removing step, a method of compression bonding a mount film (for example, a polyester film such as a PET film) whose subject surface is wetted with liquid and then peeling a porous film from the mount film after drying or without drying can be employed. Due to the surface tension of the liquid or electrostatic adhesion, the porous film is peeled from the mount film with only the surface layer of the porous film left on the mount film.

«Applications of porous film»

The porous film described above can be used as a filter. The porous film can be used for removal of fine particles or for removal of viruses, and is preferably used as a filter for removal of viruses. The filter for removal of viruses can be used either for removal of viruses in air or for removal of viruses in liquid. When the porous film is used as the filter for removal of viruses, the average diameter of the interconnected pore of the porous film is preferably 0.5 times or more and 1.5 times or less the average size of viruses to be removed.

The filter for removal of viruses can be applied to various devices. Specific examples of the device to which the filter for removal of viruses can be applied include the following devices. When viruses are removed from air, specific examples include an air purifier, an air conditioner, a gas purifier and the like. When viruses are removed from liquid, specific examples include a water purifier, a purified water-producing device and the like. Since the filter for removal of viruses can be industrially utilized, it can be utilized for producing a virus-free sample or a virus-free product in which viruses have been removed from a liquid, a gaseous sample or a product containing viruses.

(1) A porous film includes: a structure of spherical pores communicating with each other,
an interconnected pore is an opening of the spherical pores communicating with each other, and the pore diameter of the interconnected pore is 10 nm or more and 35 nm or less, and the number of the spherical pores which are present between one surface of the porous film and the other surface thereof and are 50 nm or more and 200 nm or less is 200 or more and 1000 or less.
(2) In the porous film described in (1), the porous film includes one or more selected from the group consisting of polyimide, polyethersulfone and polyvinylidene fluoride.
(3) In the porous film described in (1) or (2), the average diameter of the spherical pores is 2 times or more and 6 times or less the average diameter of the interconnected pore.
(4) In the porous film described in any one of (1) to (3), the porous film is used as a filter for removing, from a gas or a liquid containing viruses, the viruses.
(5) In the porous film described in (4), the average diameter of the interconnected pore is 0.5 times or more and 1.5 times or less the average size of the viruses.
(6) A virus removal method includes: using the porous film described in any one of (1) to (5) as a filter to remove, from a gas or a liquid containing viruses, the viruses.
(7) A method for producing a virus-free sample or a virus-free product includes: using the porous film described in any one of (1) to (5) as a filter to remove, from a liquid, a gaseous sample or a product containing viruses, the viruses.
(8) A device includes: a function of removing, from a gas or a liquid containing viruses, the viruses; and
the porous film described in any one of (1) to (5) serving as a virus removal filter.
(9) The device described in (8) is a water purifier or an air purifier.

EXAMPLES

The present invention will be described in further detail below using Examples and Comparative Examples. The present invention is not limited to these Examples.

[Preparation of varnish for producing porous film]

(Examples 1 to 4 and Comparative Examples 1 to 4)

A silica dispersion liquid (including 0.5% by mass of a dispersant relative to silica) was added into a polyamide acid solution such that the ratio of the mass of a resin component and the ratio of the mass of the silica relative to the total of the mass of polyamide acid (resin component) and the mass of the silica were ratios (mass%) shown in Table 1. Furthermore, organic solvents (1) and (2) were respectively added such that a solvent composition in the entire final composition was organic solvent (1) : organic solvent (2) = 90 : 10. The resulting mixture was added into the rotation/revolution mixer (product name: Awatori Rentaro made by Thinky Corporation), and was thereafter kneaded for 5 minutes at a rotation speed of 2000 rpm to prepare a varnish for producing a porous film having a solid content concentration of 30% by mass. A polyamide acid solution, organic solvents, a dispersant, and fine particles described below were used.
• Polyamide acid solution: a reaction product of pyromellitic dianhydride and 4,4'-diaminodiphenyl ether (solid content 200 by mass (organic solvent: N,N-dimethylacetamide))
• Organic solvent (1): N,N-dimethylacetamide (DMAc)
• Organic solvent (2): gamma butyrolactone
• Dispersant: polyoxyethylene secondary alkyl ether dispersant
• Fine particles: silica

[Table 1]

| | Resin component | Silica | |
|---|---|---|---|
| | Mass%* | Average particle size | Mass%* |
| Example 1 | 30% | 80nm | 70% |
| Example 2 | 30% | 100nm | 70% |
| Example 3 | 30% | 100nm | 70% |
| Example 4 | 30% | 100nm | 70% |
| Comparative Example 1 | 30% | 100nm | 70% |
| Comparative Example 2 | 30% | 100nm | 70% |
| Comparative Example 3 | 30% | 200nm | 70% |
| Comparative Example 4 | 30% | 300nm | 70% |
| * Mass% relative to total of mass of resin and mass of fine particles | | | |

The varnish for producing a porous film which was obtained was applied onto a polyethylene terephthalate (PET) film serving as a base material with an applicator to form an uncalcined composite film. Then, the uncalcined composite film was separated from the base material. The uncalcined composite film was placed into an oven to be calcined at 380°C for 15 minutes, and thus imidization was completed, with the result that a resin-fine particle composite film was obtained. The resin-fine particle composite film was immersed in hydrogen fluoride (HF) for 10 minutes to remove silica fine particles included in the film, and was then rinsed and dried, with the result that polyimide porous films having predetermined film thicknesses in Examples 1 to 4 and Comparative Examples 1 to 4 were obtained.

(Example 5)

A varnish for producing a porous film was prepared in the same manner as in Example 2 except that the polyamide acid was changed to polyethersulfone, the solid content concentration of the varnish was changed from 30% by mass to 35% by mass, 5% by mass of a phosphoric acid-based dispersant relative to the silica was further added into the silica dispersion liquid and only DMAc was used as the organic solvent.

The varnish for producing a porous film which was obtained was applied onto a polyethylene terephthalate (PET) film serving as a base material with an applicator. Thereafter, the coating film was baked at 50°C for 5 minutes to form a resin-fine particle composite film. The resin-fine particle composite film was immersed in water for 3 minutes. Thereafter, the resin-fine particle composite film was separated from the base material. The resin-fine particle composite film was immersed in hydrogen fluoride (HF) for 10 minutes to remove silica fine particles included in the film, and was then rinsed and dried, with the result that a polyethersulfone porous film having a predetermined film thicknesses in Example 5 was obtained.

For the porous films obtained as described above, the average diameter of a connected pore, the average diameter of spherical pores and the number of spherical pores in a film thickness direction were measured using an FIB-SEM. The results of the measurements are shown in Table 2.

[Table 2]

| | Film thickness | Average diameter of connected pore | Average diameter of spherical pores | Number of spherical pores in film thickness direction |
|---|---|---|---|---|
| Example 1 | 40μm | 20nm | 80nm | 313 pieces |
| Example 2 | 39μm | 30nm | 100nm | 402 pieces |
| Example 3 | 68μm | 30nm | 100nm | 704 pieces |
| Example 4 | 19μm | 30nm | 100nm | 201 pieces |
| Example 5 | 39μm | 25nm | 100nm | 403 pieces |
| Comparative Example 1 | 15μm | 30nm | 100nm | 152 pieces |

(continued)

| | Film thickness | Average diameter of connected pore | Average diameter of spherical pores | Number of spherical pores in film thickness direction |
|---|---|---|---|---|
| Comparative Example 2 | 118μm | 30nm | 100nm | 1207 pieces |
| Comparative Example 3 | 39μm | 50nm | 200nm | 201 pieces |
| Comparative Example 4 | 58μm | 30nm | 300nm | 204 pieces |

(Comparative Examples 5 and 6)

In Comparative Examples 5 and 6, the following porous films were used. Comparative Example 5: Porous film disclosed in Example 1 of related document 1 (the average diameter of the connected pore: 20 nm, film thickness: 40 um)

Comparative Example 6: Porous film disclosed in Example 1 of related document 3 (the average diameter of connected pore: 100 nm, film thickness: 40 um)

[Measurements of lifetime and virus leakage prevention performance]

(Test method)

Except for changing test bacteria to the following test viruses, a test was performed under the following conditions according to the "microfiltration film Ellen and module bacterial capture performance test method (JIS K 3835)". Test conditions:

- Test bacteria (viruses): Escherichia coli phage MS2 NBRC 13898 (E. coli phage*) (hereinafter referred to as MS2 viruses)
- Host bacteria was Escherichia coli NBRC 13965 (E. coli)
- Test solution: Test virus solution in which test bacteria were suspended in a phosphate buffer of pH 7.2
- Sterilization: Autoclave sterilization at 121°C for 20 minutes after setting in a stainless steel holder
- Wetting liquid: 60% 2-propanol

(Preparation of test bacteria)

The MS2 viruses described above were used as the test viruses, and thus the test bacteria were prepared according to "Japan Electrical Manufacturers' Association Project JEM1467 Household Air Purifier Appendix D Removal Performance Evaluation Test for Airborne Viruses".

(Preparation of test virus solution)

In an NB medium, a host bacterial solution obtained by culturing the host bacteria overnight at 36°C ± 2°C was inoculated with the above-described test viruses (MS2 viruses). The host bacterial solution inoculated with the test viruses was mixed with a semi-fluid agar (NB medium + 0.5% sodium chloride + 0.5% Agar). The resulting mixture was layered on a regular agar medium. The host bacteria were cultured at 36°C ± 2°C for 18 hours, and were thereafter removed by centrifugation with the agar. The liquid obtained by centrifugal removal was filtered with a surface bran filter having a pore diameter of 0.22 μm, and thus the test virus solution of about 1011 PFU/mL was obtained.

(MS2 virus removal test method)

The porous films of Examples 1 to 5 and Comparative Examples 1 to 6 were used as test filters, and thus the test virus solution was filtered. The number of viruses in 1 mL of the resulting filtrate was measured, and the virus capture performance (LRV) of the test filters was calculated by formula (a).

$$M = \log_{10} (A/B) \quad (a)$$

M: Virus capture performance (LRV)
A: Total number of test viruses added to microfiltration film
B: Total number of test viruses in the filtrate (when the number of test viruses in B is "0", B is assumed to be "1")

In the method for measuring the number of viruses in B described above, for the test virus solution (filtrate) which had passed through the test filter, a 10-fold stage dilution series was prepared with sterile purified water. This solution was mixed with the host bacterial solution and the semi-fluid agar, was layered on a regular agar medium and was thereafter cultured at 36°C ± 2°C for 48 hours. After culturing, the number of generated plaques was counted to obtain the number of viruses (PFU/mL).

[Evaluation of lifetime]

The MS2 virus removal test was performed for 10 consecutive days. At that time, a filtrate pressure during filtration was measured

The filtrate pressure on the 1st day was assumed to be $P_1$, and the filtrate pressure on the 10th day was assumed to be $P_{10}$. Based on the following formula, the rate of change in filtrate pressure was calculated. Rate of change in filtrate pressure

$$(\%) = P_{10} / P_1 \times 100$$

The lifetime was evaluated according to the following criteria based on the calculated film thickness change value. The results are shown in Table 3. A: Pressure rise was 50% or less B: Pressure rise was greater than 50% and 100% or less C: Pressure rise was greater than 100%

[Evaluation of virus leakage prevention]

The MS2 virus removal test was performed for 10 consecutive days. The number of viruses in the filtrate was calculated by formula (a). Based on the calculated number of viruses, virus leakage prevention was evaluated according to the following criteria, and the results thereof are shown in Table 3. A: LRV was 8 or less
B: LRV was greater than 8

[Table 3]

| | Lifetime | Virus leakage prevention performance |
|---|---|---|
| Example 1 | B | A |
| Example 2 | A | A |
| Example 3 | B | A |
| Example 4 | A | A |
| Example 5 | A | A |
| Comparative Example 1 | A | B |
| Comparative Example 2 | C | A |
| Comparative Example 3 | A | B |
| Comparative Example 4 | A | B |
| Comparative Example 5 | B | A |
| Comparative Example 6 | A | B |

It has been found from Table 3 that in the porous films of Examples 1 to 5, the diameter of the connected pore was 10 nm or more and 35 nm or less, and the number of spherical pores which were present between one surface of the porous film and the other surface thereof and were 50 nm or more and 200 nm or less was 200 or more and 1000 or less, and consequently, the porous films of Examples 1 to 5 had excellent lifetimes and excellent virus leakage prevention. On the other hand, it has been found that in the porous films of Comparative Examples 1 to 6, the diameter of the connected pore was 10 nm or more and 35 nm or less, and the number of spherical pores which were present

between one surface of the porous film and the other surface thereof and had a diameter of 50 nm or more and 200 nm or less was not 200 or more and 1000 or less, and consequently, it was difficult for the porous films of Comparative Examples 1 to 6 to have excellent lifetimes and excellent virus leakage prevention.

## Claims

1. A porous film comprising: a structure of spherical pores communicating with each other,

    wherein an interconnected pore is an opening of the spherical pores communicating with each other, and a pore diameter of the interconnected pore is 10 nm or more and 35 nm or less, and
    a number of the spherical pores which are present between one surface of the porous film and the other surface thereof and are 50 nm or more and 200 nm or less is 200 or more and 1000 or less.

2. The porous film according to claim 1, wherein the porous film comprises one or more selected from the group consisting of polyimide, polyethersulfone and polyvinylidene fluoride.

3. The porous film according to claim 1 or 2, wherein an average diameter of the spherical pores is 2 times or more and 6 times or less an average diameter of the interconnected pore.

4. The porous film according to claim 1 or 2, wherein the porous film is used as a filter for removing, from a gas or a liquid containing viruses, the viruses.

5. The porous film according to claim 4, wherein an average diameter of the interconnected pore is 0.5 times or more and 1.5 times or less an average size of the viruses.

6. A virus removal method comprising: using the porous film according to claim 1 or 2 as a filter to remove, from a gas or a liquid containing viruses, the viruses.

7. A method for producing a virus-free sample or a virus-free product, the method comprising: using the porous film according to claim 1 or 2 as a filter to remove, from a liquid, a gaseous sample or a product containing viruses, the viruses.

8. A device comprising: a function of removing, from a gas or a liquid containing viruses, the viruses; and
the porous film according to claim 4 serving as a virus removal filter.

9. The device according to claim 8, wherein the device is a water purifier or an air purifier.

Europäisches Patentamt
European Patent Office
Office européen des brevets

# EUROPEAN SEARCH REPORT

Application Number
EP 23 19 1409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 3 159 057 A2 (TOKYO OHKA KOGYO CO LTD [JP]) 26 April 2017 (2017-04-26)<br>* paragraphs [0180], [0185], [0247], [0344] - [0345], [0481]; figure 1 * | 1-5,8,9<br>2 | INV.<br>B01D67/00<br>B01D71/34<br>B01D71/64<br>B01D71/68 |
| X<br>Y | US 2010/155325 A1 (ZHANG LIFENG [US] ET AL) 24 June 2010 (2010-06-24)<br>* Example;<br>paragraphs [00025], [0003], [0046] - [0047]; claims 1,3,15; figure 3D * | 1,3-9<br>2 | |
| X<br>Y | CN 102 417 604 A (LANZHOU CHEM PHYS INST) 18 April 2012 (2012-04-18)<br>* Embodiment 4 Par [0004] and [0016] of enclosed machine translation * | 1-9<br>2 | |
| X<br>Y | US 4 177 228 A (PROLSS LUDWIG [CH]) 4 December 1979 (1979-12-04)<br>* column 3, line 32 - line 34; claim 13; example 1 * | 1-9<br>2 | |
| X<br>Y | US 2013/112613 A1 (KANG HYO [KR] ET AL) 9 May 2013 (2013-05-09)<br>* paragraphs [0005], [0011], [0018], [0045], [0063], [0080]; figures 1,2,3 * | 1-9<br>2 | TECHNICAL FIELDS SEARCHED (IPC)<br>B01D |
| X | EP 2 913 094 A1 (PALL CORP [US]) 2 September 2015 (2015-09-02)<br>* paragraphs [0009], [0045], [0064]; figure 2; examples 1-3 *<br>* abstract * | 1-7 | |

The present search report has been drawn up for all claims

1

| Place of search | Date of completion of the search | Examiner |
|---|---|---|
| Munich | 10 January 2024 | Williams, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 19 1409

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2024

| Patent document cited in search report | Publication date | Patent family member(s) | Publication date |
|---|---|---|---|
| EP 3159057 A2 | 26-04-2017 | CN 106552517 A | 05-04-2017 |
| | | EP 3159057 A2 | 26-04-2017 |
| | | KR 20170038712 A | 07-04-2017 |
| | | TW 201727394 A | 01-08-2017 |
| | | US 2017090293 A1 | 30-03-2017 |
| US 2010155325 A1 | 24-06-2010 | NONE | |
| CN 102417604 A | 18-04-2012 | NONE | |
| US 4177228 A | 04-12-1979 | AU 3754678 A | 03-01-1980 |
| | | CA 1107922 A | 01-09-1981 |
| | | CH 625966 A5 | 30-10-1981 |
| | | DK 293578 A | 16-01-1979 |
| | | EP 0000687 A1 | 07-02-1979 |
| | | ES 471714 A1 | 01-02-1979 |
| | | FI 782208 A | 16-01-1979 |
| | | GR 64587 B | 18-04-1980 |
| | | IL 55011 A | 20-05-1981 |
| | | IT 1097184 B | 26-08-1985 |
| | | JP S5420970 A | 16-02-1979 |
| | | PT 68212 A | 01-07-1978 |
| | | US 4177228 A | 04-12-1979 |
| | | ZA 783619 B | 27-06-1979 |
| US 2013112613 A1 | 09-05-2013 | KR 20130049616 A | 14-05-2013 |
| | | US 2013112613 A1 | 09-05-2013 |
| EP 2913094 A1 | 02-09-2015 | AU 2014277784 A1 | 17-09-2015 |
| | | CN 104874297 A | 02-09-2015 |
| | | EP 2913094 A1 | 02-09-2015 |
| | | JP 6069662 B2 | 01-02-2017 |
| | | JP 2015164718 A | 17-09-2015 |
| | | SG 10201408445T A | 29-09-2015 |
| | | US 2015246321 A1 | 03-09-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004035180 A1
- WO 2009141965 A1
- JP 2019048297 A
- JP S63283705 A
- JP H2198619 A